(19) Europäisches Patentamt
European Patent Office
Office européen des brevets

(11) Veröffentlichungsnummer: **0 260 521**
**A1**

# (12) EUROPÄISCHE PATENTANMELDUNG

(21) Anmeldenummer: **87112716.3**

(22) Anmeldetag: **01.09.87**

(51) Int. Cl.4: **C07D 231/44**, C07D 401/04, A01N 43/56, A01N 43/40

(30) Priorität: **12.09.86 DE 3631003**

(43) Veröffentlichungstag der Anmeldung: **23.03.88 Patentblatt 88/12**

(84) Benannte Vertragsstaaten: **BE CH DE FR GB IT LI NL**

(71) Anmelder: **BAYER AG**
**Konzernverwaltung RP Patentabteilung**
**D-5090 Leverkusen 1 Bayerwerk(DE)**

(72) Erfinder: **Gallenkamp, Bernd, Dr.**
**Claudiusweg 5**
**D-5600 Wuppertal 1(DE)**
Erfinder: **Wroblowsky, Heinz-Jürgen, Dr.**
**Gladbacher Strasse 34**
**D-4018 Langenfeld(DE)**
Erfinder: **Bielefeldt, Dietmar, Dr.**
**Beuthener Strasse 13**
**D-4030 Ratingen(DE)**

## (54) Verfahren zur Herstellung von 4-substituierten 1-Aryl-5-amino-pyrazolen.

(57) Es wird ein neues Verfahren zur Herstellung von teilweise bekannten, insektizid und herbizid wirksamen 4-substituierten 1-Aryl-5-amino-pyrazolen der allgemeinen Formel (I)

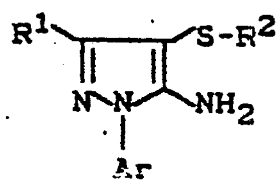

(I)

bereitgestellt,
in welcher
$R^1$ für Wasserstoff oder Alkyl steht,
$R^2$ für Alkyl oder Halogenalkyl steht und
Ar für jeweils mehrfach substituiertes Phenyl oder Pyridyl steht.
Man erhält die Verbindungen der Formel (I), wenn man Arylhydrazin-Hydrohalogenide der Formel (II),
$Ar-NH-NH_2 \times HHal$ (II)
in welcher
Hal für Halogen steht und
Ar die oben angegebene Bedeutung hat,
mit Acrylnitril-Derivaten der Formel (III),

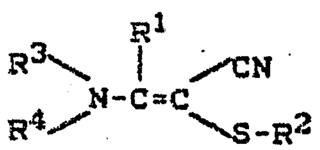

(III)



Xerox Copy Centre

in welcher
$R^1$ und $R^2$ die oben angegebene Bedeutung haben und
$R^3$ und $R^4$ unabhängig voneinander jeweils für Alkyl oder Phenyl stehen oder
$R^3$ und $R^4$ gemeinsam mit dem Stickstoffatom, an welches sie gebunden sind, für einen gesättigten Heterocyclus stehen,
gegebenenfalls in Gegenwart eines Verdünnungsmittels bei Temperaturen zwischen 60 °C und 90 °C umsetzt.

## Verfahren zur Herstellung von 4-substituierten 1-Aryl-5-amino-pyrazolen

Die Erfindung betrifft ein neues Verfahren zur Herstellung von teilweise bekannten 4-substituierten 1-Aryl-5-amino-pyrazolen, welche insektizide und herbizide Eigenschaften besitzen.

Es ist bereits bekannt, daß man bestimmte 4-substituierte 1-Aryl-5-amino-pyrazole erhält, wenn man 4-unsubstituierte 1-Aryl-5-amino-pyrazole mit elektrophilen Reagenzien umsetzt (vgl. DE-OS 34 02 308). Die Herstellung der als Vorprodukte verwendeten 4-unsubstituierten 1-Aryl-5-amino-pyrazole erfolgt dabei in der Regel aus geeignet substituierten Acrylnitril-Derivaten und entsprechenden Arylhydrazinen gegebenenfalls über intermediär zu isolierende Zwischenstufen (vgl. z. B. DE-OS 34 02 308 und DE-OS 21 41 700).

Die Nachteile einer mehrstufigen Reaktionsführung liegen auf der Hand, insbesondere wenn schlecht zugängliche und damit teure Ausgangsverbindungen - wie in diesem Fall geeignet substituierte Arylhydrazine - schon in der ersten Stufe eingesetzt werden müssen.

Weiterhin ist bekannt, daß man bestimmte 4-substituierte 1-Aryl-5-amino-pyrazole auch erhält, wenn man die entsprechenden Arylhydrazine unter bestimmten Reaktionsbedingungen direkt mit geeignet substituierten Acrylnitril-Derivaten cyclisiert (vgl. DE-OS 34 02 308).

Dieses Verfahren eignet sich jedoch nur für bestimmte in 4-Stellung einzuführende Substituenten. So gelingt es beispielsweise nicht, α-Alkylthio-oder α-Halogenalkylthio-substituierte β-Dimethyl-amino-acrylnitrile mit Arylhydrazinen direkt zu cyclisieren, ohne daß sich die Ausgangs-und/oder Endprodukte bei den erforderlichen hohen Temperaturen teilweise zersetzen, besonders wenn die Reaktivität der beteiligten Arylhydrazine durch mehrere elektronegative Substituenten stark herabgesetzt ist. Die Verwendung von α-Alkylthio-oder α-Halogenalkylthio-β-alkoxy-substituierten Acrylnitrilen scheitert schon an der schlechten Herstellbarkeit dieser Ausgangsverbindungen. Die insbesondere als Insektizide und Herbizide interessanten 4-Alkylthio-und 4-Halogenalkylthio-5-amino-1-aryl-pyrazole mit mehreren in der Regel elektronegativen Substituenten im Arylteil (vgl. DE-OS 34 02 308 bzw. die noch nicht veröffentlichte Deutsche Patentanmeldung P 35 17 843 vom 17.05.1985) sind daher auf diesem Wege nicht in befriedigender Ausbeute und Reinheit zugänglich.

Es wurde nun gefunden, daß man die teilweise bekannten 4-substituierten 1-Aryl-5-amino-pyrazole der allgemeinen Formel (I),

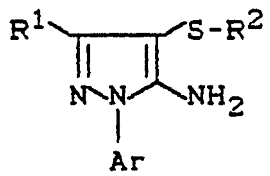

(I)

in welcher
$R^1$ für Wasserstoff oder Alkyl steht,
$R^2$ für Alkyl oder Halogenalkyl steht und
Ar für jeweils mehrfach substituiertes Phenyl oder Pyridyl steht,
erhält, wenn man Arylhydrazin-Hydrohalogenide der Formel (II),

$Ar\text{-}NH\text{-}NH_2 \times HHal$ (II)

in welcher
Hal für Halogen steht und
Ar die oben angegebene Bedeutung hat,
mit Acrylnitril-Derivaten der Formel (III),

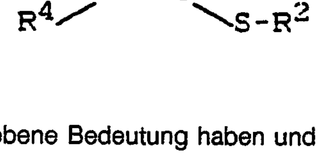

(III)

in welcher
$R^1$ und $R^2$ die oben angegebene Bedeutung haben und
$R^3$ und $R^4$ unabhängig voneinander jeweils für Alkyl oder Phenyl stehen oder

$R^3$ und $R^4$ gemeinsam mit dem Stickstoffatom, an welches sie gebunden sind, für einen gesättigten Heterocyclus stehen,
gegebenenfalls in Gegenwart eines Verdünnungsmittels bei Temperaturen zwischen 60 °C und 90 °C umsetzt.

Es ist als ausgesprochen überraschend anzusehen, daß die erfindungsgemäße Umsetzung unter diesen Reaktionsbedingungen in hoher Ausbeute durchgeführt werden kann, da aus dem Stand der Technik zu erwarten war, daß mehrfach elektronegativ substituierte Arylhydrazine erst bei wesentlich höheren Temperaturen den gewünschten Ringschluß eingehen (vgl. z. B. DE-OS 21 41 700 oder DE-OS 32 26 513 oder EP 138 149) und da die analoge Umsetzung der Acrylnitril-Derivate der Formel (III) mit freien Arylhydrazin-Basen anstelle der erfindungsgemäß verwendbaren Arylhydrazin Hydrohalogenide der Formel (II) nicht zu nennenswerten Ausbeuten an gewünschten Produkten der Formel (I) führt.

Die mit Hilfe des erfindungsgemäßen Verfahrens herstellbaren 4-substituierten 1-Aryl-5-amino-pyrazole sind durch die Formel (I) allgemein definiert. Vorzugsweise herstellbar sind Verbindungen der Formel (I), bei welchen
$R^1$ für Wasserstoff oder für geradkettiges oder verzweigtes Alkyl mit 1 bis 6 Kohlenstoffatomen steht,
$R^2$ für geradkettiges oder verzweigtes Alkyl mit 1 bis 6 Kohlenstoffatomen oder für geradkettiges oder verzweigtes Halogenalkyl mit 1 bis 4 Kohlenstoffatomen und 1 bis 9 gleichen oder verschiedenen Halogenatomen steht und
Ar für zwei-bis fünfach gleich oder verschieden substituiertes Phenyl oder für jeweils zwei-bis vierfach, gleich oder verschieden substituiertes 2-Pyridyl, 3-Pyridyl oder 4-Pyridyl steht, wobei als Substituenten jeweils in Frage kommen:
Cyano, Nitro, Halogen, jeweils geradkettiges oder verzweigtes Alkyl, Alkoxy oder Alkoxycarbonyl mit jeweils bis zu 4 Kohlenstoffatomen, außerdem jeweils geradkettiges oder verzweigtes Halogenalkyl oder Halogenalkoxy mit jeweils bis zu 4 Kohlenstoffatomen und bis zu 9 gleichen oder verschiedenen Halogenatomen oder ein Rest $-S(O)_m-R^5$,
wobei
$R^5$ für Amino, sowie für jeweils geradkettiges oder verzweigtes Alkyl, Alkylamino, Dialkylamino oder Halogenalkyl mit jeweils bis zu 4 Kohlenstoffatomen in den einzelnen Alkylteilen und im Fall des Halogenalkyl mit bis zu 9 gleichen oder verschiedenen Halogenatomen steht und
m für eine Zahl 0, 1 oder 2 steht.

Besonders bevorzugt herstellbar sind Verbindungen der Formel (I), bei welchen
$R^1$ für Wasserstoff, Methyl, Ethyl, n-oder i-Propyl, n-, i-, s-, oder t-Butyl steht,
$R^2$ für Methyl, Ethyl, n-oder i-Propyl, n-, i-, s-oder t-Butyl, für Trifluormethyl, Trichlormethyl, Difluorchlormethyl oder Dichlorfluormethyl, Fluormethyl oder Difluormethyl steht und
Ar für zwei-bis fünffach gleich oder verschieden substituiertes Phenyl oder für jeweils zwei-bis vierfach gleich oder verschieden substituiertes 2-Pyridyl oder 4-Pyridyl steht, wobei als Substituenten jeweils in Frage kommen:
Cyano, Nitro, Fluor, Chlor, Brom, Iod, Methyl, Ethyl, n-und i-Propyl, n-, i-, s und t-Butyl, Methoxy, Ethoxy, Methoxycarbonyl, Ethoxycarbonyl, Trifluormethyl, Trichlormethyl, Difluorchlormethyl, Dichlorfluormethyl, Chlormethyl, Dichlormethyl, Difluormethyl, Pentafluorethyl, Tetrafluorethyl, Tri fluorchlorethyl, Trifluorethyl, Difluordichlorethyl, Trifluordichlorethyl, Pentachlorethyl, Trifluormethoxy, Trichlormethoxy, Dichlorfluormethoxy, Difluorchlormethoxy, Chlormethoxy, Dichlormethoxy, Difluormethoxy, Pentafluorethoxy, Tetrafluorethoxy, Trifluorchlorethoxy, Trifluorethoxy, Difluordichlorethoxy, Trifluordichlorethoxy, Pentachlorethoxy oder ein Rest $-S(O)_m-R^5$, wobei
$R^5$ für Amino, Methylamino, Ethylamino, Dimethylamino, Diethylamino, Fluordichlormethyl, Difluormethyl, Tetrafluorethyl, Trifluorchlorethyl, Trifluormethyl, Methyl oder Ethyl steht und
m für eine Zahl 0, 1 oder 2 steht.

Verwendet man als Ausgangsstoffe beispielsweise 2,6-Dichlor-4-trifluormethylphenylhydrazin Hydrochlorid und 2-Trifluormethylthio-3-dimethylaminoacrylnitril, so läßt sich der Reaktionsablauf des erfindungsgemäßen Verfahrens durch das folgende Formelschema darstellen:

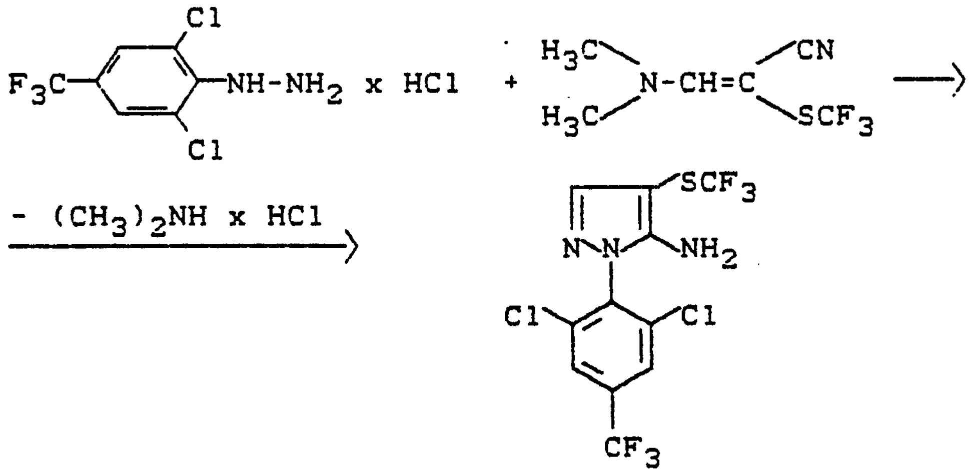


Die zur Durchführung des erfindungsgemäßen Verfahrens als Ausgangsstoffe benötigten Arylhydrazin Hydrohalogenide sind durch die Formel (II) allgemein definiert. In dieser Formel (II) steht Ar vorzugsweise für diejenigen Reste, die bereits im Zusammenhang mit der Beschreibung der erfindungsgemäßen Stoffe der Formel (I) als bevorzugt für diesen Substituenten genannt wurden. Hal steht vorzugsweise für Chlor oder Brom.

Die Arylhydrazin Hydrohalogenide der Formel (II) sind bekannt oder erhältlich in Analogie zu bekannten Verfahren, beispielsweise indem man die bekannten Arylhydrazine der Formel (IIa) (vgl. z. B. EP 138 149 oder DE-OS 34 02 308 oder DE-OS 34 47 211),

$Ar-NH-NH_2$ (IIa)

in welcher

Ar die oben angegebene Bedeutung hat,

mit Halogenwasserstoffsäuren der Formel (IV),

HHal (IV)

in welcher

Hal die oben angegebene Bedeutung hat,

gegebenenfalls in Gegenwart eines Verdünnungsmittels wie beispielsweise Ethanol zusammen gibt und Verdünnungsmit tel sowie eventuell im Überschuß vorhandene Halogenwasserstoffsäure mit üblichen Methoden (Destillation, Filtration) wieder entfernt.

Es ist auch möglich die Arylhydrazin Hydrohalogenide der Formel (II) direkt im Reaktionsansatz herzustellen, indem man als Ausgangverbindung die entsprechenden freien Arylhydrazin Basen der Formel (IIa) einsetzt und mindestens äquimolare Mengen an Halogenwasserstoffsäure der Formel (IV) dem Reaktionsansatz zusetzt.

Die zur Durchführung des erfindungsgemäßen Verfahrens weiterhin als Ausgangsstoffe benötigten Acrylnitril-Derivate sind durch die Formel (III) allgemein definiert. In dieser Formel (III) stehen $R^1$ und $R^2$ vorzugsweise für diejenigen Reste, die bereits im Zusammenhang mit der Beschreibung der erfindungsgemäßen Stoffe der Formel (I) als bevorzugt für diese Substituenten genannt wurden. $R^3$ und $R^4$ stehen unabhängig voneinander vorzugsweise für Methyl, Ethyl oder Phenyl oder gemeinsam mit dem Stickstoffatom, an welches sie gebunden sind, für einen Pyrrolidinyl-, Piperidinyl-oder Morpholinylrest.

Die Acrylnitril-Derivate der Formel (III) sind bekannt (vgl. z. B. Chem. Ber. 107, 1545 - 1554 [1974] oder Tetrahedron Lett. 22, 4259 - 4262 [1981]) oder erhältlich in Analogie zu bekannten Verfahren beispielsweise indem man die größtenteils bekannten Alkylthio-bzw. Halogenalkylthio-acetonitrile der Formel (V),

$R^2-S-CH_2-CN$ (V)

in welcher

$R^2$ die oben angegebene Bedeutung hat,

mit allgemein bekannten Aminoacetalen der Formel (VI),

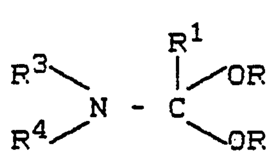 (VI)

in welcher

R für Alkyl, insbesondere für Methyl steht und

$R^1$, $R^3$ und $R^4$ die oben angegebene Bedeutung haben,

gegebenenfalls in Gegenwart eines Verdünnungsmittels oder auch ohne Verdünnungsmittel bei Temperaturen zwischen 0 °C und 60 °C umsetzt.

Als Verdünnungsmittel zur Durchführung des erfindungsgemäßen Verfahrens kommen polare organische Lösungsmittel oder wässrige Gemische in Frage. Hierzu gehören insbesondere Alkohole wie Methanol, Ethanol oder Propanol, Nitrile, wie Acetonitril oder Propionitril, Amide, wie Dimethylformamid, Dimethylacetamid, N-Methylformanilid, N-Methylpyrrolidon oder Hexamethylphosphorsäuretriamid, Sulfoxide wie Dimethylsulfoxid oder deren Gemische mit Wasser.

Die Reaktionstemperaturen können bei der Durchführung des erfindungsgemäßen Verfahrens in einem größeren Bereich variiert werde. Im allgemeinen arbeitet man bei Temperaturen zwischen 60 °C und 90 °C, vorzugsweise bei Temperaturen zwischen 70 °C und 80 °C.

Zur Durchführung des erfindungsgemäßen Verfahrens setzt man pro Mol an Arylhydrazin Hydrohalogenid der Formel (II) im allgemeinen 1 bis 1.5 Mol, vorzugsweise äquimolare Mengen an Acrylnitril-Derivat der Formel (III) ein. Die Reaktionsmischung wird für mehrere Stunden bei der erforderlichen Reaktionstemperatur gerührt und nach üblichen Verfahren aufgearbeitet, beispielsweise indem man nach Abdestillieren des Lösungsmittels das Reaktionsgemisch in Dichlormethan aufnimmt, eventuelle Verunreinigungen und Nebenprodukte durch Waschen mit Wasser entfernt, die organische Phase trocknet und im Vakuum einengt. Die Charakterisierung der so erhältlichen Produkte der Formel (I) erfolgt mit Hilfe des Schmelzpunktes oder bei nicht kristallisierenden Verbindungen mit Hilfe des Protonenkernresonanzspektrums.

Die erfindungsgemäß herstellbaren Verbindungen sind größtenteils bekannt als Herbizide (vgl. DE-OS 34 02 308) und daneben auch gute Insektizide (vgl. eigene noch nicht veröffentlichte deutsche Patentanmeldung P 35 17 843 vom 17.05.1985).

## Herstellungsbeispiele

### Beispiel 1

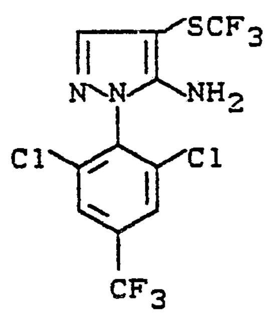

Zu einer Lösung aus 1,69 g (0.01 Mol) 2-Trifluormethyl-thio-3-dimethylaminoacrylnitril und 2,45 g (0.01 Mol) 2,6-dichlor-4-trifluormethylphenylhydrazin in 20 ml Ethanol gibt man tropfenweise 2 ml (0.07 Mol) konzentrierter Salzsäure und erhitzt für 78 Stunden auf Rückflußtemperatur. Zur Aufarbeitung engt man die Reaktionsmischung im Vakuum ein, nimmt den Rückstand in Dichlormethan auf, wäscht mit Wasser, trocknet über Natriumsulfat und entfernt das Lösungsmittel im Vakuum.

Man erhält 3,5 g (88 % der Theorie) an 5-Amino-1-(2,6-dichlor-4-trifluormethylphenyl)-4-trifluormethylthio-pyrazol vom Schmelzpunkt 113 °C - 117 °C.

Herstellung der Ausgangsverbindung

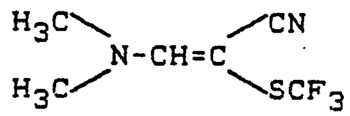

(III-1)

19,74 g (0.14 Mol) Trifluormethylthioacetonitril (vgl. J. Org. Chem. 37, 130 - 1346 [1972]) und 133 g (0.14 Mol) Dimethylformamiddimethylacetal werden 3 Stunden bei 150 °C gerührt und anschließend im Vakuum von flüchtigen Bestandteilen befreit.

Man erhält 25,5 g (100 % der Theorie) an 3-Dimethylamino-2-trifluromethylthio-acrylnitril vom Brechungsindex $n_D^{20}$ = 1.4916.

Beispiel 2

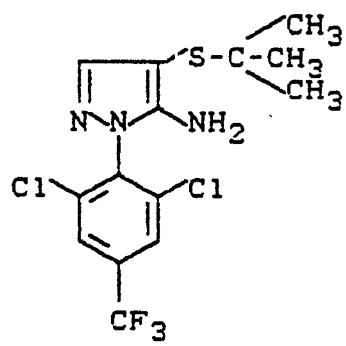

9,2 g (0.05 Mol) 1-t-Butylthio-2-dimethyl-amino-acrylnitril und 14,1 g (0.05 Mol) 2,6-Dichlor-4-trifluormethylphenylhydrazin Hydrochlorid in 250 ml Ethanol werden 2 Tage unter Rückfluß erhitzt. Zur Aufarbeitung engt man die Mischung in Vakuum ein, nimmt den Rückstand in Dichlormethan auf, wäscht mit Wasser und gesättigter wässriger Natriumchlorid-Lösung, trocknet über Magnesiumsulfat, entfernt das Lösungsmittel im Vakuum und kristallisiert den Rückstand durch Verreiben mit Petrolether.

Man erhält 12,6 g (66 % der Theorie) an 5-Amino-4-t-butylthio-1-(2,6-dichlor-4-trifluormethylphenyl)-pyrazol vom Schmelzpunkt 129 °C.

Herstellung der Ausgangsverbindung

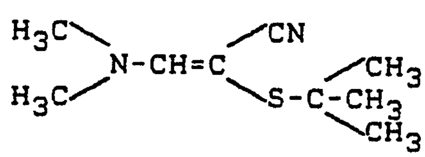

(III-2)

49,6 g (0.384 Mol) t-Butylthioacetonitril und 91,5 g (0.769 Mol) Dimethylformamiddimethylacetal werden 15 Stunden bei Raumtemperatur gerüht, im Vakuum eingeengt und im Hochvakuum fraktioniert.

Man erhält 25,2 g (36 % der Theorie) an 1-t-Butylthio-2-dimethylaminoacryl-nitril vom Siedepunkt 138 °C -145 °C bei 2 mbar. $^1$H-NMR ($CDCl_3$/TMS) : $\delta$ = 1.29; 3.17; 6.84 ppm.

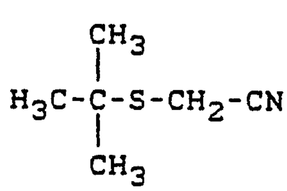

Zu 11,5 g (0.5 Mol) Natrium in 300 ml Methanol gibt man bei Raumtemperatur tropfenweise 45,1 g (0.5 Mol) t-Butylmercaptan. Nach beendeter Zugbe rührt man 30 Minuten bei Raumtemperatur nach, tropft dann 37,7 g (0.5 Mol) Chloracetonitril zu und erhitzt für 3 Stunden auf Rückflußtemperatur. Zur Aufarbeitung engt man ein, filtriert ausgefallenes Kochsalz ab und fraktioniert den Rückstand im Vakuum.

Man erhält 49,3 g (76 % der Theorie) an t-Butyl-thioacetonitril vom Siedepunkt 74 °C - 76 °C bei 8 mbar.

In entsprechender Weise und gemäß den allgemeinen Angaben zur Herstellung erhält man die folgenden 4-substituierten 1-Aryl-5-amino-pyrazole der allgemeinen Formel (I)

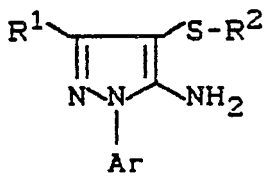

(I)

Tabelle 1

| Beisp.-Nr. | $R^1$ | $R^2$ | Ar | Schmelzpunkt [°C] |
|---|---|---|---|---|
| 3 | H | $C_2H_5$- | 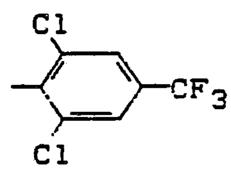 | 81 - 82 |
| 4 | H | $(CH_3)_2CH-CH_2$- | 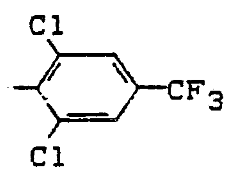 | 78 |

In entsprechender Weise und gemäß den allgemeinen Angaben zur Herstellung erhält man die folgenden Vorprodukte der allgemeinen Formel (III)

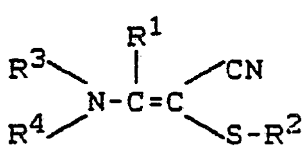

(III)

Tabelle 2

| Beisp.-Nr. | $R^1$ | $R^2$ | 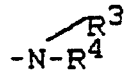 | Siedepunkt |
|---|---|---|---|---|
| III-3 | H | $C_2H_5$- | $-N(CH_3)_2$ | Kp 145-148 °C/ 8 mbar |
| III-4 | H | $(CH_3)_2CH-CH_2$- | $-N(CH_3)_2$ | Kp 142 °C/ 1 mbar |
| III-5 | H | 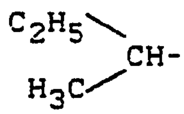 | $-N(CH_3)_2$ | Kp 134-140 °C/ 1 mbar |

## Ansprüche

1. Verfahren zur Herstellung von 4-substituierten 1-Aryl-5-amino-pyrazolen der allgemeinen Formel (I)

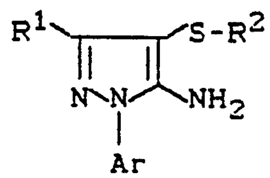

(I)

in welcher
$R^1$ für Wasserstoff oder Alkyl steht,
$R^2$ für Alkyl oder Halogenalkyl steht und
Ar für jeweils mehrfach substituiertes Phenyl oder Pyridyl steht,
dadurch gekennzeichnet, daß man Arylhydrazin-Hydrohalogenide der Formel (II),
$Ar-NH-NH_2 \times HHal$ (II)
in welcher
Hal für Halogen steht und
Ar die oben angegebene Bedeutung hat,
mit Acrylnitril-Derivaten der Formel (III),

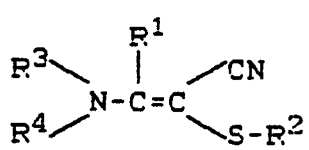

(III)

in welcher
$R^1$ und $R^2$ die oben angegebene Bedeutung haben und
$R^3$ und $R^4$ unabhängig voneinander jeweils für Alkyl oder Phenyl stehen oder
$R^3$ und $R^4$ gemeinsam mit dem Stickstoffatom, an welches sie gebunden sind, für einen gesättigten Heterocyclus stehen,
gegebenenfalls in Gegenwart eines Verdünnungsmittels bei Temperaturen zwischen 60 °C und 90 °C umsetzt.

2. Verfahren gemäß Anspruch 1 zur Herstellung von Verbindungen der Formel (I), in welcher
$R^1$ für Wasserstoff oder für geradkettiges oder verzweigtes Alkyl mit 1 bis 6 Kohlenstoffatomen steht,
$R^2$ für geradkettiges oder verzweigtes Alkyl mit 1 bis 6 Kohlenstoffatomen oder für geradkettiges oder verzweigtes Halogenalkyl mit 1 bis 4 Kohlenstoffatomen und 1 bis 9 gleichen oder verschiedenen

Halogenatomen steht und
Ar für zwei-bis fünfach gleich oder verschieden substituiertes Phenyl oder für jeweils zwei-bis vierfach, gleich oder verschieden substituiertes 2-Pyridyl, 3-Pyridyl oder 4-Pyridyl steht, wobei als Substituenten jeweils in Frage kommen:
Cyano, Nitro, Halogen, jeweils geradkettiges oder verzweigtes Alkyl, Alkoxy oder Alkoxycarbonyl mit jeweils bis zu 4 Kohlenstoffatomen außerdem jeweils geradkettiges oder verzweigtes Halogenalkyl oder Halogenalkoxy mit jeweils bis zu 4 Kohlenstoffatomen und bis zu 9 gleichen oder verschiedenen Halogenatomen oder ein Rest $-S(O)_m-R^5$,
wobei
$R^5$ für Amino, sowie für jeweils geradkettiges oder verzweigtes Alkyl, Alkylamino, Dialkylamino oder Halogenalkyl mit jeweils bis zu 4 Kohlenstoffatomen in den einzelnen Alkylteilen und im Fall des Halogenalkyl mit bis zu 9 gleichen oder verschiedenen Halogenatomen steht und
m für eine Zahl 0, 1 oder 2 steht.

3. Verfahren gemäß Anspruch 1 zur Herstellung von Verbindungen der Formel (I), in welcher
$R^1$ für Wasserstoff, Methyl, Ethyl, n-oder i-Propyl, n-, i-, s-oder t-Butyl steht,
$R^2$ für Methyl, Ethyl, n-oder i-Propyl, n-, i-, s-oder t-Butyl, für Trifluormethyl, Trichlormethyl, Difluorchlormethyl oder Dichlorfluormethyl, Fluormethyl oder Difluormethyl steht und
Ar für zwei-bis fünffach gleich oder verschieden substituiertes Phenyl oder für jeweils zwei-bis vierfach gleich oder verschieden substituiertes 2-Pyridyl oder 4-Pyridyl steht, wobei als Substituenten jeweils in Frage kommen:
Cyano, Nitro, Fluor, Clor, Brom, Iod, Methyl, Ethyl, n-und i-Propyl, n-, i-, s-und t-Butyl, Methoxy, Ethoxy, Methoxycarbonyl, Ethoxycarbonyl, Trifluormethyl, Trichlormethyl, Difluorchlormethyl, Dichlorfluormethyl, Chlormethyl, Dichlormethyl, Difluormethyl, Pentafluorethyl, Tetrafluorethyl, Trifluorchlorethyl, Trifluorethyl, Difluordichlorethyl, Trifluordichlorethyl, Pentachlorethyl, Trifluormethoxy, Trichlormethoxy, Dichlorfluormethoxy, Difluorchlormethoxy, Chlormethoxy, Dichlormethoxy, Difluormethoxy, Pentafluorethoxy, Tetrafluorethoxy, Trifluorethoxy, Difluordichlorethoxy, Trifluorchlorethoxy, Trifluordichlorethoxy, Pentachlorethoxy oder ein Rest $-S(O)_m-R^5$, wobei
$R^5$ für Amino, Methylamino, Ethylamino, Dimethylamino, Diethylamino, Fluordichlormethyl, Difluormethyl, Tetrafluorethyl, Trifluorchlorethyl, Trifluormethyl, Methyl oder Ethyl steht und
m für eine Zahl 0, 1 oder 2 steht.

4. Verfahren gemäß Anspruch 1 bis 3, dadurch gekennzeichnet, daß man die Umsetzung bei Temperaturen zwischen 70°C und 80°C durchführt.

5. Verfahren gemäß Anspruch 1 bis 4, dadurch gekennzeichnet, daß man pro Mol Arylhydrazin-Hydrohalogenid der Formel (II) 1 bis 1,5 Mol Acrylnitrilderivat der Formel (III) einsetzt.

6. Verfahren gemäß Anspruch 1 bis 5, dadurch gekennzeichnet, daß man äquimolare Mengen Arylhydrazin-Hydrohalogenid der Formel (II) und Acrylnitrilderivat der Formel (III) einsetzt.

7. Verfahren gemäß Anspruch 1 bis 6, dadurch gekennzeichnet, daß in Gegenwart eines polaren organischen Lösungsmittels gegebenenfalls im Gemisch mit Wasser gearbeitet wird.

Europäisches Patentamt

# EUROPÄISCHER RECHERCHENBERICHT

Nummer der Anmeldung

EP 87 11 2716

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| P,Y | EP-A-0 201 852 (BAYER)<br>* Seiten 24,25,28,29,38-40 *<br>--- | 1 | C 07 D 231/44<br>C 07 D 401/04<br>A 01 N 43/56<br>A 01 N 43/40 |
| P,Y | EP-A-0 234 119 (MAY & BAKER)<br>* Seiten 58,59: Anspruch 10 *<br>----- | 1 | |
| | | | **RECHERCHIERTE SACHGEBIETE (Int. Cl.4)** |
| | | | C 07 D 231/00<br>C 07 D 401/00<br>A 01 N 43/00 |

Der vorliegende Recherchenbericht wurde für alle Patentansprüche erstellt

| Recherchenort | Abschlußdatum der Recherche | Prüfer |
|---|---|---|
| DEN HAAG | 18-12-1987 | DE BUYSER I.A.F. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P0403)